# EUROPEAN PATENT SPECIFICATION

(11) **EP 4 255 542 B1**
(45) Date of publication and mention of the grant of the patent: **12.02.2025**
(21) Application number: 21823896.2
(22) Date of filing: 07.12.2021
(51) Int. Cl.: A61M 16/06, A61M 16/04, A61B 5/08

(54) **IMPROVEMENTS RELATING TO RESPIRATORY MASKS**
VERBESSERUNGEN AN ATEMMASKEN
AMÉLIORATIONS APPORTÉES À DES MASQUES RESPIRATOIRES

(30) Priority: 07.12.2020 GB 202019236
(43) Date of publication of application: 11.10.2023
(73) Proprietor: Intersurgical AG, 9490 Vaduz (LI)
(72) Inventor: MILLER, Andrew, Wokingham Berkshire RG41 2RZ (GB)
(74) Representative: Adamson Jones
(86) International application number: PCT/EP2021/084616
(87) International publication number: WO 2022/122739

(56) References cited:
- WO-A1-2016/201358
- WO-A1-2017/217264
- US-A- 5 693 944
- QUN WANG ET AL: "Preparation of modified textile possessed of absorbing and shielding characterizations", ELECTROMAGNETIC COMPATIBILITY, 2002 3RD INTERNATIONAL SYMPOSIUM ON MAY 21-24, 2002, PISCATAWAY, NJ, USA,IEEE, 21 May 2002 (2002-05-21), pages 589 - 594, XP010630687, ISBN: 978-0-7803-7277-1

## Description

The present invention concerns respiratory masks, such as may be used in the breathing assistance and/or therapy of a patient.

Respiratory masks are used to supply inhalation gases, and possibly also atomised liquids, such as drugs in solution, to the airways of a patient. In general, a gas is supplied to a respiratory cavity that is defined by the respiratory mask urged against the face of the patient, and the patient inhales the inhalation gas from this respiratory cavity. Conventional masks typically also have an inlet for the inhalation gas, and an outlet through which exhaled gas escapes the respiratory mask.

Conventional respiratory masks for breathing assistance and/or patient therapy typically comprise a mask body defining a cavity and a sealing member at the periphery of the mask body that is urged against the wearer's face, about their nose and/or mouth.

During operation, ie whilst providing breathing assistance and/or therapy to a patient, it is desirable to be able to monitor one or more characteristics of the patient's expiratory gases, such as the CO₂ concentration, in order to provide an indication of the patient's wellbeing during operation.

Conventionally, this is done by sensing the patient's expiratory gases in an exhalation tube connected to the respiratory mask, either via a respiratory monitoring unit that the exhalation tube delivers the expiratory gases to, or by placing a sensor, such as a CO₂ cuvette, in the exhalation tube.

Alternatively, in masks which vent the patient's expiratory gases to atmosphere, ie where no exhalation tube is present, such as oxygen masks, a dedicated sample tube may be connected to a spigot on the mask, to collect some of the expiratory gases before they escape, and deliver the gases to a sensor, such as a respiratory monitoring unit.

WO2016/201358 discloses a nasal ventilation mask having one or more attachment ports located adjacent to and overlying an upper lip of a patient when worn. WO2017/217264 discloses a gas sensor kit that includes a gas sensor that measures a gas concentration of an exhalation gas of a subject and a gas supply unit that supplies a therapeutic gas, supplied through a tube, to the subject.

It is an aim of the present invention to provide a respiratory mask, typically for patient therapy, which can offer an improved expiratory gas monitoring arrangement.

According to a first aspect of the invention, there is provided respiratory apparatus comprising a sensor and a respiratory mask, the respiratory mask comprising a mask body having an enclosing wall that defines an interior cavity, and the sensor having a transmitter of electromagnetic radiation and a receiver of electromagnetic radiation, wherein the enclosing wall has a sensor portion including first and second sensor windows, a portion of the interior cavity being defined between the first and second sensor windows, and the sensor being mounted relative to the sensor portion of the respiratory mask, such that electromagnetic radiation from the transmitter is transmitted, in use, through the first sensor window of the sensor portion of the enclosing wall, through the portion of the interior cavity defined between the first and second sensor windows, through the second sensor window, to the receiver, wherein the respiratory mask further comprises an inspiratory gas inlet port for delivering inspiratory gas to the wearer, and the sensor portion is spaced from the inspiratory gas inlet port.

According to a further aspect of the invention, there is provided a kit of parts comprising a sensor and a respiratory mask, the respiratory mask comprising a mask body having an enclosing wall that defines an interior cavity, and the sensor having a transmitter of electromagnetic radiation and a receiver of electromagnetic radiation, wherein the enclosing wall has a sensor portion including first and second sensor windows, a portion of the interior cavity being defined between the first and second sensor windows, and the sensor being mountable relative to the sensor portion of the respiratory mask, such that electromagnetic radiation from the transmitter is transmitted, in use, through the first sensor window of the sensor portion of the enclosing wall, through the portion of the interior cavity defined between the first and second sensor windows, through the second sensor window, to the receiver, wherein the respiratory mask further comprises an inspiratory gas inlet port for delivering inspiratory gas to the wearer, and the sensor portion is spaced from the inspiratory gas inlet port.

According to a further aspect of the invention, there is provided a respiratory mask for use with a sensor having a transmitter of electromagnetic radiation and a receiver of electromagnetic radiation, the respiratory mask comprising a mask body having an enclosing wall that defines an interior cavity, the enclosing wall having a sensor portion including first and second sensor windows, a portion of the interior cavity being defined between the first and second sensor windows, wherein the respiratory mask further comprises an inspiratory gas inlet port for delivering inspiratory gas to the wearer, and the sensor portion is spaced from the inspiratory gas inlet port.

The arrangement of the respiratory mask provides that, in use, the sensor may be mounted relative to the sensor portion of the respiratory mask, and electromagnetic radiation from the transmitter may be transmitted through the first window of the sensor portion of the enclosing wall, through the portion of the interior cavity defined between the first and second windows, through the second window, to the receiver.

The respiratory apparatus and respiratory mask according to the invention may be advantageous in that the sensor portion being formed in the enclosing wall of the mask body allows the sensor to monitor the patient's expiratory gases before they leave the mask body, reducing the delay between the gases being exhaled and the measurements being taken. In contrast, using conventional respiratory masks that are connected to a sensor via an exhalation tube or a sampling tube, expiratory gases exhaled by the wearer have to travel from the respiratory mask to the sensor, eg a respiratory monitoring unit, before they can be monitored.

Furthermore, in respiratory masks used with an exhalation tube in a breathing circuit including a gas delivery unit, such as a ventilator or anaesthesia delivery machine, the expiratory gases often cross flow paths with incoming inspiratory gases, at least briefly, before reaching the sensor, meaning that the expiratory gases can be diluted, leading to less accurate measurements. The sensor portion being formed in the enclosing wall of the mask body may therefore be further advantageous in that, in use, it allows the sensor to monitor the patient's expiratory gases almost immediately after expiration, before significant mixing with incoming inspiratory gases. This also ensures that each time the patient breathes, the expiratory gases of that breath flush the expiratory gases of the previous breath out of the sensor portion, ensuring that the sensor is monitoring expiratory gases breathed out in the most recent breath, and thus providing more accurate readings.

The sensor may be a gas sensor. In use, the wearer of the respiratory mask may inhale inspiratory gases from the interior cavity and/or exhale expiratory gases into the interior cavity. Each of the first and second sensor windows may have an interior surface and an exterior surface. The sensor may be mounted relative to the sensor portion of the respiratory mask with the transmitter disposed adjacent to the exterior surface of the first sensor window and the receiver disposed adjacent to the exterior surface of the second sensor window.

The sensor may monitor the composition of expiratory gases exhaled by the wearer in use. The sensor may be a gas composition sensor. In particular, the sensor may monitor the carbon dioxide concentration of expiratory gases exhaled by the wearer in use. The sensor may be, for example, a carbon dioxide sensor, such as an end-tidal CO₂ (EtCO₂) sensor.

The sensor may comprise a housing. The housing may accommodate the transmitter and the receiver, ie the transmitter and receiver may be disposed in the housing. The portion of the housing that accommodates the transmitter and/or the portion of the housing that accommodates the receiver may project outwardly of the remainder of the housing, such that the transmitter and the receiver are disposed adjacent to the exterior surface of the first and second sensor windows respectively, when mounted relative to the sensor portion of the respiratory mask. The transmitter and the receiver may be substantially aligned, for example such that a beam of electromagnetic radiation emitted from the transmitter will be received at the receiver. Some misalignment may be necessary to account for refraction of the electromagnetic radiation when passing through the first and second sensor windows and the at least a portion of the cavity, although this can generally be avoided by transmitting the electromagnetic radiation at a wide beam angle.

The respiratory apparatus according to the invention may be without connecting tubes and a gas delivery unit. However, in use, the respiratory apparatus according to the invention may be connected to a gas delivery unit, for example via at least one gas delivery tube arranged to deliver inspiratory gas from the gas delivery unit to the respiratory mask.

The respiratory mask may be arranged to receive the nose and/or mouth of a wearer, eg within the interior cavity of the mask body. In particular, the respiratory mask may be shaped and/or dimensioned to receive the nose and/or mouth of a wearer, eg within the interior cavity of the mask body.

The respiratory mask may be arranged to seal about the nose and/or mouth of a wearer. The respiratory mask may comprise a sealing member. The sealing member may be positioned at or around the periphery of the mask body. The sealing member may be urged against the wearer's face in use, about their nose and/or mouth, ie to provide a seal around the wearer's nose and/or mouth. The sealing member may comprise a resiliently deformable member, such as a membrane.

The respiratory mask may be a patient therapy mask, such as a continuous positive airway pressure (CPAP) mask, a non-invasive ventilation (NIV) mask, an aerosol mask, an oxygen mask, or any other respiratory mask for delivering respiratory gases to a wearer. Alternatively, the respiratory mask may be for protective purposes. For example, the respiratory mask may be a filter mask having a filter through which ambient air passes before being inhaled by the wearer. The respiratory mask may therefore be any face mask in which monitoring of the expiratory gases exhaled by the wearer may be beneficial or desirable.

The wearer of the mask may be a patient. The inspiratory gas inlet port may be arranged to connect to a gas inlet, or have a gas inlet integrally or detachably connected thereto. The respiratory mask may comprise an expiratory gas outlet port for delivering expiratory gas from the wearer. Escape of expiratory gases via the expiratory gas outlet port may be controlled by one or more exhalation valve. The expiratory gas outlet port may be arranged to connect to a gas outlet, or have a gas outlet integrally or detachably connected thereto.

The inspiratory gas inlet port and the expiratory gas outlet port may be separate ports, ie the inspiratory gas inlet port may comprise a first aperture in the enclosing wall of the mask body and the expiratory gas outlet port may comprise a second aperture in the enclosing wall of the mask body. Alternatively, the inspiratory gas inlet port and the expiratory gas outlet port may be the same port, and the port may be operated using a Y-piece connector and/or a suitable valve arrangement, ie the inspiratory gas inlet port and the expiratory gas outlet port may comprise a single aperture in the enclosing wall of the mask body. In a further alternative, the expiratory gas outlet may comprise at least one opening in the mask body, eg a plurality of openings in the mask body. In this embodiment, escape of expiratory gases via the plurality of openings may also be controlled by exhalation valves.

The respiratory mask itself will also differ in structure relative to conventional respiratory masks.

The sensor portion may be formed separately from the expiratory gas outlet. For example, the sensor portion may be spaced from the expiratory gas outlet. This may be further advantageous in that, in use, it further reduces the chance of the patient's expiratory gases mixing with incoming inspiratory gases before measurements are taken by the sensor.

In contrast to the inspiratory gas inlet port and/or the expiratory gas outlet, the sensor portion may not be a port or an aperture. The sensor portion may be a discontinuity in the enclosing wall of the mask body. For example, the sensing portion may project, either inwardly or outwardly, relative to the enclosing wall of the mask body. The sensing portion projecting relative to the enclosing wall of the mask body and including the first and second sensor windows may be advantageous in that it makes the first and second sensor windows more accessible to the transmitter and the receiver of the sensor in use.

The electromagnetic radiation transmitted by the transmitter and received by the receiver may be infrared radiation. Infrared radiation may refer to radiation having a wavelength between approximately 700 nm and approximately 1 mm.

The first and second sensor windows may be transmissive of the electromagnetic radiation transmitted by the transmitter. For example, the first and second sensor windows may be transmissive of infrared radiation, ie radiation having a wavelength between approximately 700 nm and approximately 1 mm. The first and second sensor windows may also be transmissive to other wavelengths of light, such as visible light. The first and second sensor windows may be more transmissive of the electromagnetic radiation transmitted by the transmitter than the remainder of any of, or any combination of, the mask body, the enclosing wall of the mask body, and the sensor portion.

The first and second sensor windows may be transmissive to at least 50%, 60%, 70%, 80%, 90% or 100% of the electromagnetic radiation transmitted by the transmitter. To obtain an accurate and/or viable reading, the sensor may require that a minimum amount of electromagnetic radiation, or a minimum proportion of the electromagnetic radiation transmitted by the transmitter, is received by the receiver. Hence, the first and second sensor windows may be transmissive of the electromagnetic radiation transmitted by the transmitter such that at least 20%, 30%, 40%, 50%, 60%, 70%, 80% or 90% of the electromagnetic radiation is received at the receiver after passing through the portion of the interior cavity defined between the first and second sensor windows.

The first and second sensor windows may be of a reduced thickness relative to the remainder of any of, or any combination of, the mask body, the enclosing wall of the mask body, and the sensor portion. The first and second sensor windows may have a thickness of up to 0.5mm, up to 0.4mm, up to 0.3 mm, up to 0.2 mm, up to 0.1 mm, or up to 0.05 mm. The thickness of the first and second sensor windows may refer to the distance between the interior surface and the exterior surface of each sensor window.

The first and second sensor windows, eg the interior surface of the first and second sensor windows, may be spaced apart by a distance of between 2mm and 50mm, or between 5mm and 30mm, ie the portion of the interior cavity defined between the first and second sensor windows may have a thickness of between 2mm and 50mm, or between 5mm and 30mm.

The mask body may comprise a nose cavity portion. The nose cavity portion may comprise the inspiratory gas inlet port for delivering inspiratory gas to the wearer. The mask body may comprise a mouth cavity portion. The mouth cavity portion may comprise the sensor portion.

The enclosing wall of the mask body may have an external surface that forms the exterior of the mask body in use, ie the external surface of the mask body may face away from the wearer in use. The sensor portion may be formed integrally with any of, or any combination of, the mask body, the enclosing wall of the mask body and the external surface of the enclosing wall. The sensor portion may be formed of the same material as any of, or any combination of, the mask body, the enclosing wall of the mask body and the external surface of the enclosing wall.

The sensor portion may project or protrude outwardly from the mask body, eg from the mouth cavity portion of the mask body. The sensor portion may be substantially cuboidal, for example having the form of a cuboid or a rectangular cuboid. The sensor portion may comprise a pair of side walls that extend outwardly from the external surface of the mask body, ie away from the wearer of the mask in use, and a front wall that extends between the pair of side walls.

The pair of side walls may be angled relative to the external surface of the mask body. The pair of side walls may extend from the external surface of the mask body substantially perpendicularly to a tangential plane of the external surface of the mask body. The side walls may extend substantially perpendicularly to a frontal plane of a wearer of the respiratory mask, in use. The side walls may extend from the external surface of the mask body substantially parallel to each other.

Substantially perpendicular may comprise, for example, at an angle of between 75 and 105 degrees, or at angle of between 85 and 95 degrees. Substantially parallel may comprise, for example, at an angle of up to 15 degrees, or at angle of up to 5 degrees.

Alternatively, the sensor portion may project or protrude inwardly from the mask body, eg from the mouth cavity portion of the mask body. In this embodiment, the sensor portion may comprise a pair of side walls that extend inwardly from the external surface of the mask body, ie towards a wearer of the mask in use. In this embodiment, the sensor portion may also comprise a recess positioned either side of the pair of side walls, in which the transmitter and receiver of the sensor are positioned in use. The pair of side walls may extend inwardly from the external surface of the mask body substantially perpendicularly to a tangential plane of the external surface of the mask body. The side walls may extend inwardly substantially perpendicularly to a frontal plane of a wearer of the respiratory mask, in use. The side walls may extend inwardly from the external surface of the mask body substantially parallel to each other.

The first and second sensor windows may be formed in the pair of side walls of the sensor portion. The first sensor window may be formed in the first side wall of the sensor portion and the second sensor window may be formed in the second side wall of the sensor portion. Thus, the first and second sensor windows may extend inwardly or outwardly from the external surface of the mask body substantially perpendicularly to a tangential plane of the external surface of the mask body, and/or the first and second sensor windows may extend inwardly or outwardly from the external surface of the mask body substantially perpendicularly to a frontal plane of a wearer of the respiratory mask, in use, and/or the first and second sensor windows may extend inwardly or outwardly from the external surface of the mask body substantially parallel to each other.

The sensor portion may face the mouth of the wearer in use. The mouth cavity portion of the mask body may be arranged generally opposite the mouth of a wearer in use. The sensor portion may be formed in the mask body in a region of intersection between an expiratory flow from a wearer's nose and an expiratory flow from a wearer's mouth. The sensor portion may be formed in the mouth cavity portion. The mouth cavity portion may be substantially linear in a transverse plane of the wearer of the respiratory mask, in use. The mouth cavity portion may be curved in a longitudinal plane of the wearer of the respiratory mask, in use.

The sensor portion may be arranged to receive and/or retain the sensor. The sensor portion may comprise at least one abutment surface arranged to receive and/or retain the sensor in use. The at least one abutment surface may be shaped and/or dimensioned to receive and/or retain the sensor in use. The at least one abutment surface may be shaped and/or dimensioned so as to correspond to at least one abutment surface of the sensor. In use, the sensor may detachably or releasably engage with the sensor portion, for example with a friction fit or a snap fit. In use, the at least one abutment surface of the sensor may detachably or releasably engage with the at least one abutment surface of the sensor portion, for example with a friction fit or a snap fit.

Alternatively, a different portion of the mask body, for example a portion adjacent to the mouth cavity portion, such as the nose cavity portion, may be arranged to receive and/or retain the sensor.

The mask body may be formed by injection moulding. The first and second sensing windows may be formed in the mask body prior to cooling of the mask body, for example using hot stamping or pressing of the material using pins. Alternatively, the first and second sensing windows may be formed by any conventional methods known in the art. For example, using two-shot injection moulding, the first and second sensing windows may be formed in a first shot using a first material, and the mask body may be overmoulded in a second shot using a second material, as described in WO2008/055478. The first and second materials may be the same material, or different materials. In another example, the mask body may be formed by injection moulding, and the first and second sensing windows may be formed separately of a thin film-like material, and fitted into the mask body, as described in US5693944.

The inspiratory gas inlet port in the nose cavity portion may be arranged to direct inspiratory gas away from the mouth cavity portion. The nose cavity portion may be arranged towards a first end of the mask body and the mouth cavity portion may extend from the nose cavity portion towards an opposing end of the mask body. The first end of the mask body may be an upper end of the mask body, as viewed when worn by a wearer in use, and the opposing end of the mask body may be a lower end of the mask body, as viewed when worn by a wearer in use. The inspiratory gas inlet port may be arranged to direct inspiratory gas towards the first end.

The nose cavity portion may comprise an intervening wall depending outwardly from the mouth cavity portion, relative to a wearer in use, so as to define a nose cavity region of greater depth than that of the mouth cavity region. The inspiratory gas inlet port may be provided in the intervening wall.

The mask body may comprise a generally rigid polymer shell formed as a unitary piece and shaped to define the nose and mouth cavity portions. The inspiratory gas inlet port may extend through the rigid polymer shell between an interior and an exterior surface thereof. The mask body may be formed of a thermoplastic polymer such as polypropylene, polyethylene, polymethylmethacrylate (PMMA), or polycarbonate. Alternatively, the first and second sensor windows may be formed of a thermoplastic polymer such as polypropylene, polyethylene, polymethylmethacrylate (PMMA), or polycarbonate, and the remainder of any of, or any combination of, the mask body, the enclosing wall of the mask body, and the sensor portion may be formed of an alternative material. This may be advantageous in that thermoplastic polymers such as polypropylene and polyethylene are naturally transmissive to infrared light.

Practicable embodiments of the invention are described in further detail below by way of example only with reference to the accompanying drawings, of which:

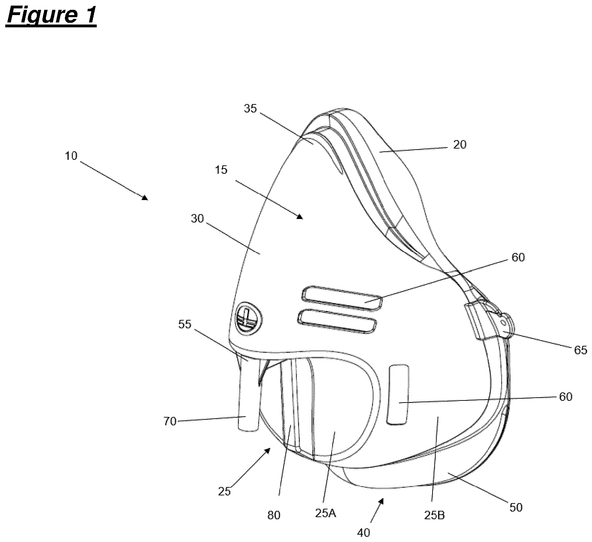

Figure 1 shows a three-dimensional view of a respiratory mask according to an example of the invention;

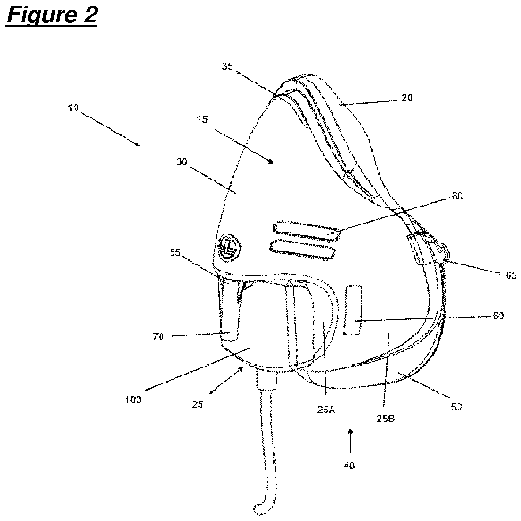

Figure 2 shows the respiratory mask of Figure 1 releasably attached to an expiratory monitoring sensor;
Figure 3 shows a rear view of the respiratory mask of Figure;
Figure 4 shows a side view of the respiratory mask; and
Figure 5 shows a side view of the respiratory mask of Figure 1 releasably attached to an expiratory monitoring sensor.

In each of the Figures there is shown a respiratory mask 10, which is suitable for the delivery of respiratory gases, such as oxygen, to a wearer, such as a patient. The respiratory mask comprises a mask body 15, often referred to as a mask shell, formed from a suitably strong and relatively rigid plastics material, in this example polypropylene, and a sealing formation 20 formed from a more flexible or compliant material, such as an elastomer. In particular, a Styrene-EthyleneButylene-Styrene (SEBS)-based thermoplastic elastomer may be used for the sealing formation. However, it will be appreciated that other conventional mask body and seal materials may be used.

The respiratory mask 10 is manufactured using a so-called two-shot injection moulding process. In particular, the mask body 15 is firstly injection moulded as a single component, the sealing formation 20 is then injection moulded onto the surface of the mask body 15, and the mask body 15 and the sealing formation 20 are bonded together by this process. However, it will be appreciated that other conventional manufacturing processes may be used.

The mask body 15 is generally dome-shaped, so as to define a cavity via which an inhalation gas is delivered to a patient, and comprises a mouth portion 25 and a nose portion 30. The mask body 15 is shaped such that the maximum depth of the cavity defined by the nose portion 30 is greater than the depth of the cavity defined by the mouth portion 25. The nose portion 30 is generally tapered towards an apex 35 at a first end of the mask 10 that is shaped to fit around the bridge of the patient's nose in use.

The mouth portion 25 generally comprises a forward-facing, front wall 25A and laterally-extending side wall portions 25B, which are arranged to be located adjacent a wearer's cheeks or jowls, and particularly the lower portion thereof, in use.

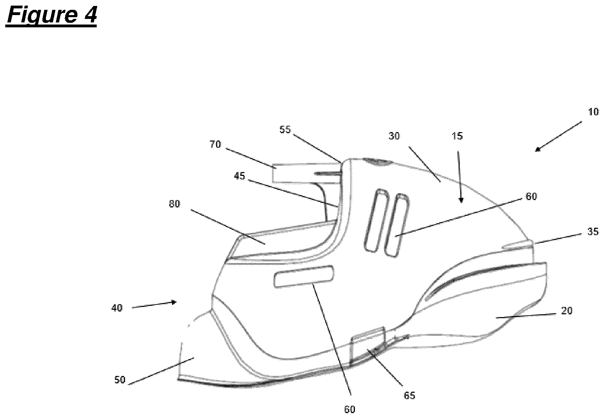

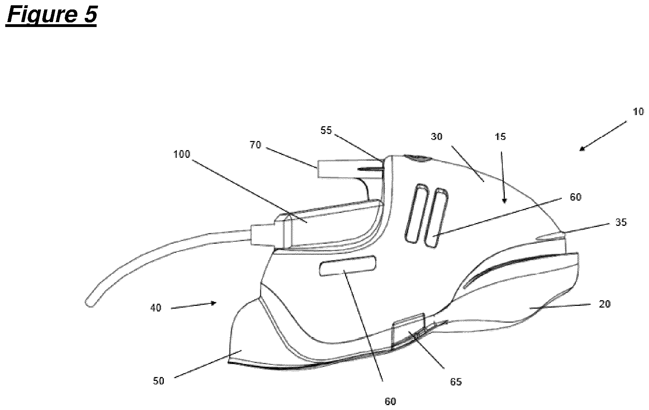

An intermediate wall portion 45 (see Figures 4 and 5) is arranged between the mouth 25 and nose 30 portions of the mask body 15 and effectively defines an interface between those portions. The intermediate wall 45 is in the form of a shelf or shoulder, for example, which projects forwardly of the front wall 25A of the mouth portion 25, relative to the wearer of the mask 10 in use. The intermediate wall 45 is angled relative to the front wall 25A, typically approximately perpendicularly. The intermediate wall 45 defines a lower wall of the nose portion 30 which projects beyond, or overhangs, the mouth portion 25.

The sealing formation 20 is a unitary flange member that is bonded to, and extends from, the peripheral edge of the mask body 15. The sealing formation 20 may pass substantially around the entire periphery of the mask body 15 and may comprise an inwardly depending lip portion, which extends into the opening defined by the edge of the mask body 15. The sealing formation 20 may have discontinuities therein, in the form of slits which allow the seal to deform about the different contour portions of a wearer's face, in use. In this example the sealing formation 20 also comprises a chin cup formation 50, which may provide a seal beneath the wearer's chin, in use, particularly for wearer's having a larger facial length.

The elastomeric nature of the sealing formation 20 enables an effective seal to be formed between the contact surface of the respiratory mask 10 and the face of the patient in use. However, it will be appreciated that the mask 10 may adopt different sealing formations about its peripheral edge in line with other conventional mask designs.

Furthermore, it is possible that the provision of a second, more-flexible sealing material 20 may be omitted altogether where the seal quality is of little consequence to the mask provider.

The mask body 15 further comprises an inlet port 55 for connection to a supply of an inhalation gas, such as oxygen. The inlet port 55 comprises an opening in the intermediate wall 45 (i.e. in a lower wall of the nose portion 30), and a tubular connector 70 that extends outwardly/downwardly away from the mask body 15 into the space in front of the mouth portion 25. The free end of the connector 70 is thus disposed outside of the mask body 15 in front of the mouth portion 25, and the inlet port 55 and the connector 70 are spatially separated from the front face 25A of the mouth portion 25. In use, a supply of an inhalation gas is connected to the tubular connector 70 of the inlet port 55 via a supply tube so as to supply the inhalation gas to the cavity of the respiratory mask 10 and hence the airways of the patient.

The mask body 15 has one or more exhalation openings 60, which may be spaced from the inlet opening 55. In this embodiment the exhalation openings are simple apertures in the wall of the mask body 15 that allow exhaled gases to exit the cavity of the respiratory mask 10. The exhalation openings 60 are elongate in form. A pair of exhalation openings 60 is provided to either side of the nose portion 30. A generally vertically aligned exhalation opening 60 is also provided on either side of the front face 25A of the mouth portion 25 (i.e. in side walls 25B). It will be appreciated that other shapes, configurations and orientations of exhalation openings 60 are possible. In some embodiments, the exhalation openings 60 may comprise a simple valve structure.

The mask body 15 has a pair of outwardly extending flange formations 65 on either side of the respiratory mask 10 which are arranged to receive an elastic strap in use. Each flange 65 is located adjacent the peripheral edge of the mask body 15 and has an aperture, to which an elastic strap (not shown in the Figures) is attached, in use.

The elastic strap extends between the flanges 65, and fits around the patient's head when the respiratory mask 10 is fitted to the patient. In use, the strap is adjusted so that the respiratory mask 10 is urged against the face of the patient with an appropriate force to ensure that an effective seal is formed between the periphery of the respiratory mask 10 and the wearer's face, without causing excessive discomfort for the wearer.

In use, the mask 10 is urged against a wearer's face such that the first end (ie the apex 35) is uppermost and rests against the bridge of the wearer's nose, typically at, or slightly below, the nasion, and the second end 40 is located towards or beneath the wearer's mouth, typically in the vicinity of the chin, such that generally the wearer's nose and mouth are located in the mask cavity, but in the case of patient's having a larger face, at least the wearer's nose is located in the mask cavity. The wearer's mouth is accommodated within the mouth portion 25 of the mask body 15 and the wearer's nose is accommodated within the nose portion 30 of the mask body 15. The nose portion 30 is tapered towards the upper end 35 of the mask 10 and hence the bridge of the patient's nose.

The front face 25A of the mouth portion 25 comprises a generally cuboidal protrusion 80 that extends forwardly of the front wall 25A of the mouth portion 25, relative to the wearer of the mask 10 in use. The protrusion 80 comprises two side walls and a front face, the two side walls extending forwardly of the front wall 25A of the mouth portion 25, relative to the wearer of the mask 10 in use, and the front face extending between the two side walls.

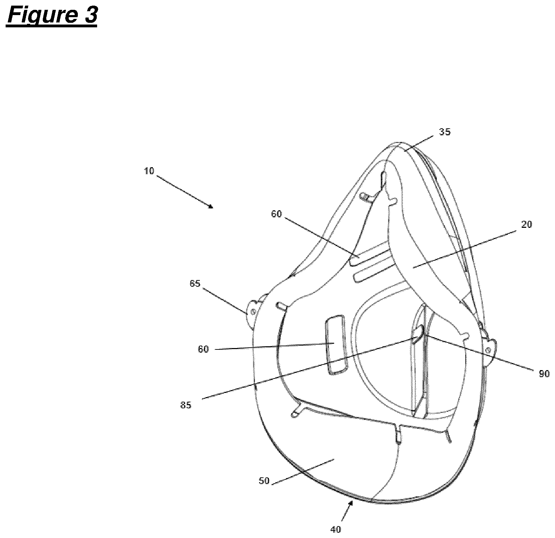

Each of the side walls are substantially opposite one another, and comprise a window portion 85 (see Figure 3). Although only one window portion 85 can be seen in Figure 3, due to the angle of the illustration, the window portions 85 are positioned substantially opposite one another within the respective side walls, ie at substantially the same position in the side walls. Each of the window portions 85 is transmissive at least of infrared light, such that the space 90 formed between the window portions 85 may serve as an expiratory gas monitoring portion. The expiratory gas monitoring portion 90 is substantially in front of, and diametrically opposite, the wearer's mouth, in use.

The front face 25A of the mouth portion 25, and the protrusion 80 in particular, is further arranged to function as a receiving port, such that a sensor 100, eg an EtCO₂ sensor, may be releasably attached to the protrusion 80 of the mask 10, to enable a measurement of the expiratory gas within the expiratory gas monitoring portion 90 to be taken. In particular, the protrusion 80 is shaped and dimensioned to correspond with the shape and dimensions of an engaging portion (not shown) of the sensor 100, such that the sensor 100 may be clipped onto the mask 10 by engaging with the protrusion 80, for example with a friction fit or a snap fit. It is foreseen that alternative conventional retention mechanisms may be employed for engaging the sensor with the front face 25A of the mouth portion 25.

In use, gas is supplied to the mask interior via the inlet 55, thereby generally flooding the nose portion 15 at least. During inspiration, gas within the interior of the mask is drawn in via the nose and/or mouth. In the event that the rate at which gas is drawn into the wearer's lungs is greater the gas supply rate via the inlet 55, additional ambient air will be drawn into the mask via openings 60.

During expiration, the wearer will breathe out via their nose and/or their mouth, generally flooding the expiratory gas monitoring portion 90 with expiratory gas. The attached sensor 100 comprises an emitter and a receiver, and emits, via the emitter, an infrared signal that travels into the expiratory gas monitoring portion 90 through a first of the window portions 85, and out of the expiratory gas monitoring portion 90 through the other of the window portions 85, to be received, via the receiver. Based on the proportion of infrared that is received at the receiver, it is possible to determine the amount of carbon dioxide that is present in the expiratory gas, for example via comparison with known infrared absorption spectrums, and thus detect any abnormalities in the breathing functionality of the wearer.

## Claims

1. Respiratory apparatus comprising a sensor (100) and a respiratory mask (10), the respiratory mask (10) comprising a mask body (15) having an enclosing wall that defines an interior cavity, and the sensor (100) having a transmitter of electromagnetic radiation and a receiver of electromagnetic radiation, wherein the enclosing wall has a sensor portion including first and second sensor windows (85), a portion of the interior cavity (90) being defined between the first and second sensor windows (85), and the sensor (100) being mounted relative to the sensor portion of the respiratory mask (10), such that electromagnetic radiation from the transmitter is transmitted, in use, through the first window of the sensor portion of the enclosing wall, through the portion of the interior cavity (90) defined between the first and second windows (85), through the second window, to the receiver, wherein the respiratory mask (10) further comprises an inspiratory gas inlet port (55) for delivering inspiratory gas to the wearer, and the sensor portion is spaced from the inspiratory gas inlet port (55).

2. A kit of parts comprising a sensor (100) and a respiratory mask (10), the respiratory mask (10) comprising a mask body having an enclosing wall that defines an interior cavity, and the sensor (100) having a transmitter of electromagnetic radiation and a receiver of electromagnetic radiation, wherein the enclosing wall has a sensor portion including first and second sensor windows (85), a portion of the interior cavity (90) being defined between the first and second sensor windows (85), and the sensor (100) being mountable relative to the sensor portion of the respiratory mask (10), such that electromagnetic radiation from the transmitter is transmitted, in use, through the first sensor window of the sensor portion of the enclosing wall, through the portion of the interior cavity (90) defined between the first and second sensor windows (85), through the second sensor window, to the receiver, wherein the respiratory mask (10) further comprises an inspiratory gas inlet port (55) for delivering inspiratory gas to the wearer, and the sensor portion is spaced from the inspiratory gas inlet port (55).

3. Respiratory apparatus according to Claim 1 or a kit according to Claim 2, wherein the sensor (100) is a gas composition sensor.

4. Respiratory apparatus or a kit according to any preceding claim, wherein each of the first and second sensor windows (85) have an interior surface and an exterior surface, and the sensor is mounted relative to the sensor portion of the respiratory mask (10) with the transmitter disposed adjacent to the exterior surface of the first sensor window and the receiver disposed adjacent to the exterior surface of the second sensor window.

5. Respiratory apparatus or a kit according to Claim 4, wherein the sensor (100) comprises a housing which accommodates the transmitter and the receiver, and the portion of the housing that accommodates the transmitter and the portion of the housing that accommodates the receiver both project outwardly of the remainder of the housing, such that the transmitter is disposed adjacent to the exterior surface of the first sensor window and the receiver is disposed adjacent to the exterior surface of the second sensor window.

6. A respiratory mask (10) for use with a sensor having a transmitter of electromagnetic radiation and a receiver of electromagnetic radiation, the respiratory mask comprising a mask body (15) having an enclosing wall that defines an interior cavity, the enclosing wall having a sensor portion including first and second sensor windows (85), a portion of the interior cavity (90) being defined between the first and second sensor windows (85), wherein the respiratory mask (10) further comprises an inspiratory gas inlet port (55) for delivering inspiratory gas to the wearer, and the sensor portion is spaced from the inspiratory gas inlet port (55).

7. Respiratory apparatus, a kit or a respiratory mask (10) according to any preceding claim, wherein the respiratory mask (10) further comprises an expiratory gas outlet port (60) for delivering expiratory gas from the wearer, the expiratory gas outlet port (60) being arranged to connect to a gas outlet, or having a gas outlet integrally or detachably connected thereto, and wherein the sensor portion is formed separately and/or is spaced from the expiratory gas outlet (60).

8. Respiratory apparatus, a kit or a respiratory mask (10) according to any preceding claim, wherein the sensor portion is formed separately from the inspiratory gas inlet port (55).

9. Respiratory apparatus, a kit or a respiratory mask (10) according to any preceding claim, wherein the sensor portion is a discontinuity in the enclosing wall of the mask body (15), and/or wherein the enclosing wall of the mask body (15) has an external surface that forms the exterior of the mask body (15), and the sensing portion projects, either inwardly or outwardly, relative to the external surface of the mask body (15).

10. Respiratory apparatus, a kit or a respiratory mask (10) according to any preceding claim, wherein the first and second sensor windows (85) are transmissive of the electromagnetic radiation transmitted by the transmitter, and/or wherein the first and second sensor windows (85) are more transmissive of the electromagnetic radiation transmitted by the transmitter than the remainder of any of, or any combination of, the mask body, the enclosing wall of the mask body (15), the external surface of the mask body (15), and the sensor portion.

11. Respiratory apparatus, a kit or a respiratory mask (10) according to any preceding claim, wherein the mask body (15) comprises a mouth cavity portion (25) and the sensor portion is formed in the mouth cavity portion (25).

12. Respiratory apparatus, a kit or a respiratory mask (10) according to any preceding claim, wherein the mask body (15) further comprises a nose cavity portion (30) and the inspiratory gas inlet port (55) is formed in the nose cavity portion (30).

13. Respiratory apparatus, a kit or a respiratory mask (10) according to any preceding claim, wherein the sensor portion is formed in the mask body (15) in a region of intersection between an expiratory flow from a wearer's nose and an expiratory flow from a wearer's mouth.

14. Respiratory apparatus, a kit or a respiratory mask (10) according to any preceding claim, wherein the enclosing wall of the mask body (15) has an external surface that forms the exterior of the mask body, and the sensor portion comprises a pair of side walls that are angled relative to the external surface of the mask body (15), wherein the first and second sensor windows (85) are formed in the pair of side walls.

15. Respiratory apparatus, a kit or a respiratory mask (10) according to any preceding claim, wherein the respiratory mask (10) is for protective purposes and/or is a filter mask.

## Patentansprüche

1. Atemgerät, umfassend einen Sensor (100) und eine Atemmaske (10), wobei die Atemmaske (10) einen Maskenkörper (15) mit einer umschließenden Wand umfasst, die einen inneren Hohlraum definiert, und der Sensor (100) einen Sender für elektromagnetische Strahlung und einen Empfänger für elektromagnetische Strahlung aufweist, wobei die umschließende Wand einen Sensorabschnitt einschließlich einem ersten und einem zweiten Sensorfenster (85) aufweist, wobei ein Abschnitt des inneren Hohlraums (90) zwischen dem ersten und dem zweiten Sensorfenster (85) definiert ist und wobei der Sensor (100) relativ zu dem Sensorabschnitt der Atemmaske (10) montiert ist, sodass elektromagnetische Strahlung von dem Sender bei Gebrauch durch das erste Fenster des Sensorabschnitts der umschließenden Wand, durch den Abschnitt des inneren Hohlraums (90), der zwischen dem ersten und dem zweiten Fenster (85) definiert ist, durch das zweite Fenster an den Empfänger übertragen wird, wobei die Atemmaske (10) ferner eine Einatemgaseinlassöffnung (55) zum Zuführen von Einatemgas an den Träger umfasst und der Sensorabschnitt von der Einatemgaseinlassöffnung (55) beabstandet ist.

2. Kit aus Teilen, umfassend einen Sensor (100) und eine Atemmaske (10), wobei die Atemmaske (10) einen Maskenkörper mit einer umschließenden Wand umfasst, die einen inneren Hohlraum definiert, und der Sensor (100) einen Sender für elektromagnetische Strahlung und einen Empfänger für elektromagnetische Strahlung aufweist, wobei die umschließende Wand einen Sensorabschnitt einschließlich einem ersten und einem zweiten Sensorfenster (85) aufweist, wobei ein Abschnitt des inneren Hohlraums (90) zwischen dem ersten und dem zweiten Sensorfenster (85) definiert ist und wobei der Sensor (100) relativ zu dem Sensorabschnitt der Atemmaske (10) montierbar ist, sodass elektromagnetische Strahlung von dem Sender bei Gebrauch durch das erste Sensorfenster des Sensorabschnitts der umschließenden Wand, durch den Abschnitt des inneren Hohlraums (90), der zwischen dem ersten und dem zweiten Sensorfenster (85) definiert ist, durch das zweite Sensorfenster an den Empfänger übertragen wird, wobei die Atemmaske (10) ferner eine Einatemgaseinlassöffnung (55) zum Zuführen von Einatemgas an den Träger umfasst, und wobei der Sensorabschnitt von der Einatemgaseinlassöffnung (55) beabstandet ist.

3. Atemgerät nach Anspruch 1 oder Kit nach Anspruch 2, wobei der Sensor (100) ein Gaszusammensetzungssensor ist.

4. Atemgerät oder Kit nach einem der vorhergehenden Ansprüche, wobei das erste und das zweite Sensorfenster (85) jeweils eine Innen- und eine Außenfläche aufweisen und der Sensor relativ zum Sensorabschnitt der Atemmaske (10) montiert ist, wobei der Sender neben der Außenfläche des ersten Sensorfensters angeordnet ist und der Empfänger neben der Außenfläche des zweiten Sensorfensters angeordnet ist.

5. Atemgerät oder Kit nach Anspruch 4, wobei der Sensor (100) ein Gehäuse umfasst, das den Sender und den Empfänger aufnimmt, und sowohl der Abschnitt des Gehäuses, der den Sender aufnimmt, als auch der Abschnitt des Gehäuses, der den Empfänger aufnimmt, über den Rest des Gehäuses nach außen vorstehen, sodass der Sender neben der Außenfläche des ersten Sensorfensters angeordnet ist und der Empfänger neben der Außenfläche des zweiten Sensorfensters angeordnet ist.

6. Atemmaske (10) zur Verwendung mit einem Sensor, der einen Sender für elektromagnetische Strahlung und einen Empfänger für elektromagnetische Strahlung aufweist, wobei die Atemmaske einen Maskenkörper (15) mit einer umschließenden Wand umfasst, die einen inneren Hohlraum definiert, wobei die umschließende Wand einen Sensorabschnitt einschließlich einem ersten und einem zweiten Sensorfenster (85) aufweist, wobei ein Abschnitt des inneren Hohlraums (90) zwischen dem ersten und dem zweiten Sensorfenster (85) definiert ist, wobei die Atemmaske (10) ferner eine Einatemgaseinlassöffnung (55) zum Zuführen von Einatemgas an den Träger umfasst und der Sensorabschnitt von der Einatemgaseinlassöffnung (55) beabstandet ist.

7. Atemgerät, Kit oder Atemmaske (10) nach einem der vorhergehenden Ansprüche, wobei die Atemmaske (10) ferner eine Ausatemgasauslassöffnung (60) zum Abgeben von Ausatemgas vom Träger umfasst, wobei die Ausatemgasauslassöffnung (60) dazu arrangiert ist, mit einem Gasauslass verbunden zu werden, oder einen Gasauslass aufweist, der einstückig oder abnehmbar damit verbunden ist, und wobei der Sensorabschnitt separat ausgebildet ist und/oder von dem Ausatemgasauslass (60) beabstandet ist.

8. Atemgerät, Kit oder Atemmaske (10) nach einem der vorhergehenden Ansprüche, wobei der Sensorabschnitt separat von der Einatemgaseinlassöffnung (55) ausgebildet ist.

9. Atemgerät, Kit oder Atemmaske (10) nach einem der vorhergehenden Ansprüche, wobei der Sensorabschnitt eine Unterbrechung in der umschließenden Wand des Maskenkörpers (15) ist und/oder wobei die umschließende Wand des Maskenkörpers (15) eine Außenfläche aufweist, die die Außenseite des Maskenkörpers (15) bildet, und der Sensorabschnitt relativ zur Außenfläche des Maskenkörpers (15) entweder nach innen oder nach außen vorsteht.

10. Atemgerät, Kit oder Atemmaske (10) nach einem der vorhergehenden Ansprüche, wobei das erste und das zweite Sensorfenster (85) für die vom Sender ausgesandte elektromagnetische Strahlung durchlässig sind und/oder wobei das erste und das zweite Sensorfenster (85) für die vom Sender ausgesandte elektromagnetische Strahlung durchlässiger als der Rest von jedem von dem Maskenkörper, der umschließenden Wand des Maskenkörpers (15), der Außenfläche des Maskenkörpers (15) und dem Sensorabschnitt oder einer beliebigen Kombination davon sind.

11. Atemgerät, Kit oder Atemmaske (10) nach einem der vorhergehenden Ansprüche, wobei der Maskenkörper (15) einen Mundhöhlenabschnitt (25) umfasst und der Sensorabschnitt im Mundhöhlenabschnitt (25) ausgebildet ist.

12. Atemgerät, Kit oder Atemmaske (10) nach einem der vorhergehenden Ansprüche, wobei der Maskenkörper (15) ferner einen Nasenhohlraumabschnitt (30) umfasst und die Einatemgaseinlassöffnung (55) im Nasenhohlraumabschnitt (30) ausgebildet ist.

13. Atemgerät, Kit oder Atemmaske (10) nach einem der vorhergehenden Ansprüche, wobei der Sensorabschnitt im Maskenkörper (15) in einem Schnittbereich zwischen einem Ausatemstrom aus der Nase eines Trägers und einem Ausatemstrom aus dem Mund eines Trägers ausgebildet ist.

14. Atemgerät, Kit oder Atemmaske (10) nach einem der vorhergehenden Ansprüche, wobei die umschließende Wand des Maskenkörpers (15) eine Außenfläche aufweist, die die Außenseite des Maskenkörpers bildet, und der Sensorabschnitt ein Paar Seitenwände umfasst, die relativ zur Außenfläche des Maskenkörpers (15) angewinkelt sind, wobei das erste und das zweite Sensorfenster (85) in dem Paar Seitenwände ausgebildet sind.

15. Atemgerät, Kit oder Atemmaske (10) nach einem der vorhergehenden Ansprüche, wobei die Atemmaske (10) Schutzzwecken dient und/oder eine Filtermaske ist.

## Revendications

1. Appareil respiratoire comprenant un capteur (100) et un masque respiratoire (10), le masque respiratoire (10) comprenant un corps de masque (15) comportant une paroi enveloppante qui définit une cavité intérieure, et le capteur (100) comportant un émetteur de rayonnement électromagnétique et un récepteur de rayonnement électromagnétique, dans lequel la paroi enveloppante comporte une partie de capteur comprenant des première et seconde fenêtres de capteur (85), une partie de la cavité intérieure (90) étant définie entre les première et seconde fenêtres de capteur (85), et le capteur (100) étant monté par rapport à la partie de capteur du masque respiratoire (10), de telle sorte que le rayonnement électromagnétique provenant de l'émetteur soit transmis, lors de l'utilisation, à travers la première fenêtre de la partie de capteur de la paroi enveloppante, à travers la partie de la cavité intérieure (90) définie entre les première et seconde fenêtres (85), à travers la seconde fenêtre, au récepteur, dans lequel le masque respiratoire (10) comprend en outre un orifice d'entrée de gaz inspiratoire (55) pour délivrer du gaz inspiratoire au porteur, et la partie de capteur est espacée de l'orifice d'entrée de gaz inspiratoire (55).

2. Kit de pièces comprenant un capteur (100) et un masque respiratoire (10), le masque respiratoire (10) comprenant un corps de masque comportant une paroi enveloppante qui définit une cavité intérieure, et le capteur (100) comportant un émetteur de rayonnement électromagnétique et un récepteur de rayonnement électromagnétique, dans lequel la paroi enveloppante comporte une partie de capteur comprenant des première et seconde fenêtres de capteur (85), une partie de la cavité intérieure (90) étant définie entre les première et seconde fenêtres de capteur (85), et le capteur (100) pouvant être monté par rapport à la partie de capteur du masque respiratoire (10), de telle sorte que le rayonnement électromagnétique provenant de l'émetteur soit transmis, en cours d'utilisation, à travers la première fenêtre de capteur de la partie de capteur de la paroi enveloppante, à travers la partie de la cavité intérieure (90) définie entre les première et seconde fenêtres de capteur (85), à travers la seconde fenêtre de capteur, au récepteur, dans lequel le masque respiratoire (10) comprend en outre un orifice d'entrée de gaz inspiratoire (55) pour délivrer du gaz inspiratoire au porteur,
et la partie capteur est espacée de l'orifice d'entrée de gaz inspiratoire (55).

3. Appareil respiratoire selon la revendication 1 ou kit selon la revendication 2, dans lequel le capteur (100) est un capteur de composition de gaz.

4. Appareil respiratoire ou kit selon l'une quelconque des revendications précédentes, dans lequel chacune des première et seconde fenêtres de capteur (85) comportent une surface intérieure et une surface extérieure, et le capteur est monté par rapport à la partie de capteur du masque respiratoire (10) avec l'émetteur disposé adjacent à la surface extérieure de la première fenêtre de capteur et le récepteur disposé adjacent à la surface extérieure de la seconde fenêtre de capteur.

5. Appareil respiratoire ou kit selon la revendication 4, dans lequel le capteur (100) comprend un boîtier qui reçoit l'émetteur et le récepteur, et la partie du boîtier qui reçoit l'émetteur et la partie du boîtier qui reçoit le récepteur font toutes deux saillie vers l'extérieur du reste du boîtier, de telle sorte que l'émetteur soit disposé adjacent à la surface extérieure de la première fenêtre de capteur et que le récepteur soit disposé adjacent à la surface extérieure de la seconde fenêtre de capteur.

6. Masque respiratoire (10) destiné à être utilisé avec un capteur comportant un émetteur de rayonnement électromagnétique et un récepteur de rayonnement électromagnétique, le masque respiratoire comprenant un corps de masque (15) comportant une paroi enveloppante qui définit une cavité intérieure, la paroi enveloppante comportant une partie de capteur comprenant des première et seconde fenêtres de capteur (85), une partie de la cavité intérieure (90) étant définie entre les première et seconde fenêtres de capteur (85), dans lequel le masque respiratoire (10) comprend en outre un orifice d'entrée de gaz inspiratoire (55) pour délivrer du gaz inspiratoire au porteur, et la partie de capteur est espacée de l'orifice d'entrée de gaz inspiratoire (55).

7. Appareil respiratoire, kit ou masque respiratoire (10) selon l'une quelconque des revendications précédentes, dans lequel le masque respiratoire (10) comprend en outre un orifice de sortie de gaz expiratoire (60) pour délivrer du gaz expiratoire en provenance du porteur, l'orifice de sortie de gaz expiratoire (60) étant agencé pour se connecter à une sortie de gaz, ou comportant une sortie de gaz connectée de manière intégrée ou détachable à celui-ci, et dans lequel la partie de capteur est formée séparément et/ou est espacée de la sortie de gaz expiratoire (60).

8. Appareil respiratoire, kit ou masque respiratoire (10) selon l'une quelconque des revendications précédentes, dans lequel la partie de capteur est formée séparément de l'orifice d'entrée de gaz inspiratoire (55).

9. Appareil respiratoire, kit ou masque respiratoire (10) selon l'une quelconque des revendications précédentes, dans lequel la partie de capteur est une discontinuité dans la paroi enveloppante du corps de masque (15), et/ou dans lequel la paroi enveloppante du corps de masque (15) comporte une surface externe qui forme l'extérieur du corps de masque (15), et la partie de détection fait saillie, soit vers l'intérieur, soit vers l'extérieur, par rapport à la surface externe du corps de masque (15).

10. Appareil respiratoire, kit ou masque respiratoire (10) selon l'une quelconque des revendications précédentes, dans lequel les première et seconde fenêtres de capteur (85) transmettent le rayonnement électromagnétique transmis par l'émetteur, et/ou dans lequel les première et seconde fenêtres de capteur (85) transmettent davantage le rayonnement électromagnétique transmis par l'émetteur que le reste de l'un quelconque, ou l'une quelconque combinaison, du corps de masque, de la paroi enveloppante du corps de masque (15), de la surface externe du corps de masque (15) et de la partie de capteur.

11. Appareil respiratoire, kit ou masque respiratoire (10) selon l'une quelconque des revendications précédentes, dans lequel le corps de masque (15) comprend une partie de cavité buccale (25) et la partie de capteur est formée dans la partie de cavité buccale (25).

12. Appareil respiratoire, kit ou masque respiratoire (10) selon l'une quelconque des revendications précédentes, dans lequel le corps de masque (15) comprend en outre une partie de cavité nasale (30) et l'orifice d'entrée de gaz inspiratoire (55) est formé dans la partie de cavité nasale (30).

13. Appareil respiratoire, kit ou masque respiratoire (10) selon l'une quelconque des revendications précédentes, dans lequel la partie de capteur est formée dans le corps de masque (15) dans une région d'intersection entre un flux expiratoire provenant du nez d'un porteur et un flux expiratoire provenant de la bouche d'un porteur.

14. Appareil respiratoire, kit ou masque respiratoire (10) selon l'une quelconque des revendications précédentes, dans lequel la paroi enveloppante du corps de masque (15) comporte une surface externe qui forme l'extérieur du corps de masque, et la partie de capteur comprend une paire de parois latérales qui sont inclinées par rapport à la surface externe du corps de masque (15), dans lequel les première et seconde fenêtres de capteur (85) sont formées dans la paire de parois latérales.

15. Appareil respiratoire, kit ou masque respiratoire (10) selon l'une quelconque des revendications précédentes, dans lequel le masque respiratoire (10) est destiné à des fins de protection et/ou est un masque filtrant.
